# EUROPEAN PATENT APPLICATION

(11) **EP 4 035 705 A1**
(43) Date of publication of application: **03.08.2022**
(21) Application number: 21154263.4
(22) Date of filing: 29.01.2021
(51) Int. Cl.: A61M 1/16, A61M 1/28

(54) **DIALYSIS SYSTEM AND METHOD**

(71) Applicant: Bellco S.r.l., 41037 Mirandola (MO) (IT); Medtronic, Inc., Minneapolis, MN 55432 (US)
(72) Inventor: KUMAR, Vijay P, Minneapolis, MN Minnesota 55432-5604 (US); PETRUCCI, Alberto, 41037 Modena (IT)
(74) Representative: Maschio, Antonio

(57) **Abstract**

A peritoneal dialysis machine that includes a preparator (60) as well as a cycler (100) to form the peritoneal dialysis system. The system delivers purified water into one or more containers (50) with different powders to create a concentrate and then moves this concentrate to a mixing bag (PDF-GEN) to create the peritoneal dialysis fluid (PDF). The cycler then delivers fresh PDF to the patient (200) and removes waste fluid via the drain outlet. The containers have unique data tags (1, 2, 3) containing container identification indicia and the machine includes integrated reading technology to retrieve the data from each container.

## Description

### FIELD

The present invention relates to a dialysis system and method, particularly but not exclusively a peritoneal dialysis system and method.

### BACKGROUND

Dialysis is a procedure to remove waste products and excess fluid from the blood when the kidneys stop working properly. One common type of dialysis is haemodialysis where blood is pumped out of a patient's body to a machine where it is passed through a series of tiny tubes, in an 'artificial kidney' or 'dialyser'. The tubes form a special membrane that allows waste products and fluid to pass across it. Clear fluid called 'dialysate' is passed outside the tubes in the opposite direction and, as the blood passes in one direction and the dialysate in the other, waste products and excess water pass into the dialysate, which is then pumped out of the machine and discarded down a drain. The cleaned blood is pumped back into the patient's body. Blood passes continuously through the dialyser during each dialysis session.

Another type of dialysis treatment is peritoneal dialysis (PD) which uses the lining of a patient's abdomen, the peritoneum, to filter blood while its remains inside the body. Peritoneal Dialysis is an effective way to treat patients with End Stage Renal Disease and acute patients with renal failure. The patient is provided with a catheter that includes a transfer set for selective connection to a dialysis solution or a drain bag. A dialysis solution ('dialysate' or 'peritoneal dialysis fluid') comprising water with other electrolytes is warmed to body temperature and then introduced into the peritoneal cavity of the patient via the catheter. The solution remains here for a period of time required to clean the blood of waste products, toxins and excess fluid and these are then removed from the body to leave the right amounts of electrolytes and nutrients in the blood. The machine or 'cycler' may repeat the exchange process a number of times (cycles), as required for a particular patient.

Conventionally dialysate consists of purified water, glucose and electrolytes, with the concentration of electrolytes resembling that which occurs naturally in the blood. It is prepared according to an individual patient's needs to help regulate their electrolyte and acid-base balance and remove metabolic waste products. The dialysate components are normally shipped in the form of concentrates, with basic and acidic concentrates being prepared separately. The basic concentrate is usually supplied as a dry concentrate consisting of pure sodium bicarbonate which is made up into a saturated solution and the acidic concentrate is usually provided as a liquid concentrate. If it is provided as a dry concentrate, the entire concentrate must be dissolved before being diluted to form the dialysate. This requires a large volume of liquid and multiple components for providing the dialysis fluid, increasing the time required for production of the fluid. Concentrate may also be wasted during the production of the correct dialysis fluid and difficulties may be encountered in providing the correct concentration of electrolytes in the fluid for a particular patient. Thus, while it is preferable to provide the concentrates in dry form to reduce their volume for shipping and storage purposes, providing the correct dialysate for a particular patient on a continuous basis can prove problematic. As a result, multiple bags of dialysate such as peritoneal dialysis fluid (PDF) are normally provided pre-prepared for dialysis. The high weights of peritoneal dialysis fluid (PDF) bags reduces the appeal of this procedure, as does the number, volume and weight of supplies required for each daily treatment (up to 15-18 Liters every day).

The filling of concentrates in powdered form or in lactate form to the bag at the manufacturing facility is prone to errors. It is practically not possible to eliminate all manufacturing errors introduced by the concentrate dispensing system during manufacture. Any error introduced by the manufacturing dispensing system in filling the concentrate will inevitably introduce the error into the reconstituted concentrate and finally into the proportioned PD fluid. In accordance with pharmacopeia standards, accuracy of ions and sodium content in the final PD fluid should be within +/-2.5% and dextrose accuracy of dextrose should be within +/-5%. This can be difficult to achieve in view of any manufacturing errors that may be present in the original concentrates.

Furthermore, the concentrate bags need to be used within the expiry date of the bag. Manual errors in reading the expiry date could lead to patient safety issues.

The ability to ensure the correct dialysis fluid is prepared and/or delivered to the patient is also open to user error and can present a manual burden. It is desirable to be able to collect and monitor information in relation to the concentrates, dialysate constituents and/or dialysis fluid that are supplied to a patient.

It is an object of the present invention to provide an improved dialysis system and method, particularly but not exclusively a peritoneal dialysis system and method, that aims to overcome or at least alleviate the above-mentioned drawbacks.

### SUMMARY

A first aspect of the present invention provides a dialysis system comprising a fluid pumping system for forming fluid paths between a container of dialysis fluid, a dialyser and to drain wherein the fluid system is provided with at least one flow generator, a readable data tag associated with the container of dialysis fluid, the tag including container identification indicia and a reader to read data from the tag.

The dialyser may comprise an artificial kidney for receiving dialysis blood that is pumped out of a patient's body wherein the blood passes through a membrane ("the dialyser") that allows waste products and fluid to pass across it. The dialysis fluid is passed outside the membrane in the opposite direction and, as the blood passes in one direction and the dialysate in the other, waste products and excess water pass into the dialysis fluid, which is then discarded to drain. The cleaned blood is pumped back into the patient's body.

The readable data tag preferably includes data relating to the content of the container. For example, the container identification indicia may include at least one of the following information: content; expiry date; weight and concentration. The reader may intermittently or continuously read this data and store the data locally or remotely. Preferably, the at least one tag is a read-write tag.

The dialysis system is provided with appropriate reading technology such as barcode, RFID tag or NFC (near field communication) reader integrated into the system to enable it to read the information stored on the data tag and use this feedback to adjust an operating mode of the system. Preferably, the system includes a data collection computer system to store this information. Alternatively, a separate handheld reader may be provided that may be include a communication link to the dialysis system, for example via Bluetooth.

The data tag may comprise any suitable tag that may be attached or associated with the container and read by a reader integrated with the dialysis system. Examples of tags include a one or two-dimensional barcode, a passive integrated transponder tag or a miniature radiofrequency tag. The system preferably includes a microprocessor which may be configurable via the dialysis system or an external host to selectively provide reading and/or writing of the data tag. The data retrieved may be stored and/or compared against other data stored in a database and the microprocessor may adjust operation of the dialysis machine dependent on the data received, such as terminating dialysis should the data state that PD fluid contained in the container is out of date.

Preferably, the tag including container identification indicia is applied by a manufacturer on each concentrate bag. For example, during the concentrate filling process, manufacturing equipment is configured to take the weight of an empty bag and the bag once filled after dispensing of the concentrate into the bag. A data writer records the difference in weight onto the data tag of the respective bag which corresponds to the weight of the concentrate in the bag. The manufacturing equipment may also write other information such as date of manufacture, expiry date, serial no, LOT number, contents of the bag etc into the tag fixed to the bag.

Preferably, the dialysis system has means to determine the actual weight of the bag provided for dialysis, such as appropriate weighing means, such as scales or a load cell. The difference between the data provided for a manufactured concentrate bag and the actual weight of the bag may be utilised to improve the accuracy of the PD fluid generated. In this respect, the system uses the concentrate weight data to calculate an accurate amount of water to be mixed with the concentrate to get the required dilution with minimal or no errors. Thus, the present invention provides an overall reduction in error of ions, dextrose and sodium content in the final PD fluid and ensures that the parameters are kept within the pharmacopeia limits.

In a preferred embodiment, the dialysis system comprises a peritoneal dialysis system wherein a patient's abdomen, the peritoneum, filters blood while it remains inside the body. In this embodiment, the patient's abdomen forms the dialyser membrane and peritoneal dialysis fluid (PDF) is warmed to body temperature and then introduced into the peritoneal cavity of the patient via a catheter. The at least one data tag including container identification indicia may be associated with the container of PDF.

More preferably still, the peritoneal dialysis system comprises a peritoneal dialysis preparator and cycler, the preparator comprising a purified water source fluidly connectable to a plurality of containers each containing one or more dialysate constituents for dissolution and proportioning to form the peritoneal dialysis fluid for delivery to the cycler for subsequent delivery to, and drainage from, a patient. In this preferred embodiment, each container preferably has a readable data tag including container identification indicia associated with it providing information of the content of that particular container, such as the content; expiry date; weight and concentration.

In a preferred embodiment, each container is fluidly connectable to a chamber for receiving a solution of the dialysate constituents for preparation of the dialysis fluid, said chamber being fluidly connectable to the cycler. At least one hydraulic flow generator may be provided in a fluid path between the containers and the chamber and/or the chamber and the cycler.

The dialysate constituents may be powders or solutes, such as substance dissolved in a solution. The powder or solute may comprise one or more chemicals or compounds. Solutes as used herein, before being dissolved in a solvent, may exist as entirely or partially dried, gels, concentrated liquids, pellets or powders, or any combination of these forms.

Preferably, the system according to the invention is provided with an arrangement of hydraulic flow or pressure generators, such as flexible pumps, throughout the fluid paths to provide optimized dissolution of the solutes, optimized mixing of the solution and/or cycling of the peritoneal dialysis fluid thus formed. It is preferable for at least one of the hydraulic flow generators to be a variable flow generator. The different configurations of hydraulic flow/pressure generators are provided in the fluid paths to provide optimization of the flow of water through the fluid system to create the correct powder concentrates and subsequent peritoneal dialysis fluid for cycling. More preferably still, the data retrieved from the data tags relating to the content of the dialysate constituent containers enables automatic adjustment of the flow generators to provide a desired PDF concentration and flow as required for a particular dialysis patient.

At least one hydraulic flow generator is preferably provided in the cycler fluid path for controlled movement of the prepared peritoneal dialysis fluid. Preferably, a hydraulic flow generator is also provided in a fluid path between the water source and the solute containers and/or the PDF chamber.

Optionally, each dialysate constituent or solute container may have a hydraulic flow generator in its fluid path with the water source. In one embodiment, three dialysate constituent containers are provided that are each fluidly connectable to the purified water source, with each fluid path having a hydraulic flow generator. This allows a peritoneal dialysis fluid to be generated without a mixing chamber prior to delivery to a patient. Again, each container has at least one data tag for transmitting container identification indicia to a data collection system and the system preferably includes a microprocessor which may be configurable via the dialysis system or an external host to selectively provide reading and/or writing of the data tags. The data retrieved may be stored and/or compared against other data stored in a database and the microprocessor may adjust operation of the dialysis system dependent on the data received, such as adjusting the flow of purified water to each solute or constituent container to alter the concentration of each reconstituted solute for mixing to form the PDF. This enables in line and on-demand production of the PD fluid, prepared according to an individual patient's need to enable regulation of their electrolyte and acid-base balance.

Preferably, the microprocessor reads a manufacturer recorded weight of each container containing one or more dialysate constituents provided on their data tag and compares this with an actual weight of each container, whereby the microprocessor applies the actual concentrate weight data to calculate and adjust the volume of purified water to be supplied to the concentrate for correct dissolution for proportioning to form the peritoneal dialysis fluid.

Any suitable hydraulic flow generator may be provided, such as an actuator operable on the fluid line. A pump, such as a hydraulic piston pump or a peristaltic pump may form the hydraulic flow generator. The hydraulic flow generators may be provided within tubing extending between the component parts of the system but more preferably are included in a cassette operable within the dialysis system. A cassette may be provided in the preparator part of the system and/or the cycler part of the system. More preferably, a cassette is provided for at least the cycler part of the system. The cassette has appropriate inlet and outlet connections, fluid paths between the inlet and outlet connections, at least one flow generator and a plurality of valves provided in the fluid paths.

The cassette may be provided with other components that are conventional in the art and therefore will not be discussed here in any detail. Such components include, *inter alia,* a casing surrounding the fluid paths, pump regions, for example, being formed by chambers open to one side that are covered by a flexible plastic film and valve regions that may block the fluid paths. The cassette may also be provided with a heating region, such as heating elements provided in a part of the cassette that surrounds a fluid path. Sensors may also be provided within the cassette to monitor fluid flow and properties, such as temperature.

It is to be appreciated that the inlets and outlets of the cassette may be connected to other fluid paths by hose elements.

The at least one container is preferably a bag that contains one or more solutes or dialysate constituents, preferably in a concentrated form and provided in separate compartments. These concentrated solutes may for example be in the form of powder, pellets, super concentrated liquid and/or gel. If a compartmentalised bag is provided as the container, preferably a data tag containing container identification indicia is associated with each compartment of the bag. The bag preferably includes a nozzle attachment to aid mixing of the solute with the purified water.

The invention described herein may further comprise a water purification unit configured to be fluidly connectable to the purified water source. The water purification unit may purify water taken from any source, such as mains water from a domestic tap or faucet.

A second aspect of the present invention provides a method of monitoring a dialysis system having fluid paths for fluid flow between a container of dialysis fluid, a dialyser and a drain, the method comprising the steps of: attaching at least one readable data tag including container identification indicia to the container and reading the data from the tag.

Preferably, the method includes storing the data retrieved from the tag and optionally comparing the data with data in a database. More preferably, the method includes adjustment of the fluid flow through the fluid paths on the basis of the retrieved data.

More preferably, the method includes retrieving data from multiple readable data tags provided on multiple containers having different solutes or dialysate constituents that are reconstituted and proportioned to form the dialysis fluid. Preferably, the method includes adjustment of fluid flow through the fluid paths to and from these containers on the basis of the retrieved data. Preferably, adjustment of the fluid flow is provided by adjustment of a flow generator and/or valve. More preferably, the method includes retrieving data relating to a manufacturer recorded weight of the container provided on the readable data tags and comparing this data with an actual weight of each container and adjusting the fluid flow to and from these containers based on this comparison for correct proportioning to form the peritoneal dialysis fluid.

### Brief description of the figures

**Figure 1** is a schematic drawing illustrating an automated peritoneal dialysis machine according to the prior art;
**Figure 2** is a block diagram of an automated peritoneal dialysis preparator and cycler machine for use with the present invention;
**Figure 3** is a Fluid Flow diagram for a dialysis fluid generator according to one embodiment of the present invention;
**Figure 4** is a Fluid Flow diagram for a dialysis fluid generator according to another embodiment of the present invention; and
**Figure 5** is a Fluid Flow diagram for a dialysis fluid generator according to yet another embodiment of the present invention.

### DETAILED DESCRIPTION

### Definitions

Unless defined otherwise, all technical and scientific terms used generally have the same meaning as commonly understood by one of ordinary skill in the art.

The articles "a" and "an" are used to refer to one or to over one (*i.e.,* to at least one) of the grammatical object of the article. For example, "an element" means one element or over one element.

The term "bag" as used herein refers to a container, and preferably a flexible, collapsible and/or foldable container. Preferably a bag is a container for fluids, most preferably for liquids.

The term "comprising" includes, but is not limited to, whatever follows the word "comprising." Use of the term indicates the listed elements are required or mandatory but that other elements are optional and may be present.

The term "concentrated" as used herein means, of a substance or solution, present in a high proportion relative to other substances, preferably having had fluid, such as water or other diluting agents, removed or reduced.

The term "consisting of" includes and is limited to whatever follows the phrase "consisting of." The phrase indicates the limited elements are required or mandatory and that no other elements may be present.

The term "consisting essentially of" includes whatever follows the term "consisting essentially of" and additional elements, structures, acts or features that do not affect the basic operation of the apparatus, structure or method described.

The term "dialysate" describes a fluid into or out of which solutes from a fluid to be dialyzed diffuse through a membrane. An initial dialysate used for therapy typically contains electrolytes close in concentration to the physiological concentration of electrolytes found in blood. However, the concentration of the dialysate can change over the course of therapy, and can further be adjusted as desired.

The term "dialysis flow path" refers to a fluid pathway or passageway configured to convey a fluid, such as dialysate and/or blood, wherein said pathway forms at least part of, preferably the whole of, a fluid circuit for peritoneal dialysis, hemodialysis, hemofiltration, hemodiafiltration or ultrafiltration. A dialysis machine may be included within the flow path. A dialyzer may be included in the flow path.

The term "dissolution" as used herein refers to the process of a solute becoming incorporated into a solvent to make a solution. Dissolution means the same as "dissolving" and the terms are used interchangeably herein.

The term "dissolution time" as used herein refers to the time taken for one or more solutes to fully dissolve in a solvent to make solution. Dissolution times of the same solutes can be compared if the conditions of dissolution (including for example solvent type and volume, temperature, flow speed of solvent and container shape) are the same.

The term "fluid" can be any substance without a fixed shape that yields easily to external pressure such as a gas or a liquid. Specifically, the fluid can be water, preferably purified water, and can be water containing any solutes at any concentration. The fluid can be used as a solvent (to dissolve one or more solutes). The fluid can also be dialysate of any type including fresh, partially used, or spent. The fluid can also contain one or more dissolved gas. The fluid may be blood.

The term "fluidly connectable" refers to the ability to provide passage of fluid from one point to another point. The ability to provide such passage can be any mechanical connection, fastening, or forming between two points to permit the flow of fluid, gas, or combinations thereof. The two points can be within or between any one or more of compartments, modules, systems, components, and rechargers, all of any type. Most preferably the connections are provided by tubes or pipes.

The term "fluidly connected" refers to a particular state or configuration of one or more components such that fluid can flow from one point to another point. The connection state can also include an optional unconnected state or configuration, such that the two points are disconnected from each other to discontinue flow. It will be further understood that the two "fluidly connectable" points, as defined above, can from a "fluidly connected" state. The two points can be within or between any one or more of compartments, modules, systems, components, and rechargers, all of any type.

The term "formed" or "formed of" as used herein refers to a material comprising any of the materials, elements, composited or compounds listed.

The term "inlet" can refer to a portion of a component through which fluid and/or gas can be drawn into a component, conduit, or fluid passageway of any type, such as a bag, container, conduit, tube or pipe of any type.

The term "measuring" or "to measure" can refer to determining any parameter or variable. The parameter or variable can relate to any state or value of a system, component, fluid, gas, or mixtures of one or more gases or fluids.

The term "measurement" refers to data or information of the parameter or variable determined.

The term "mix" means to combine one or more substance so that the resulting mixture is not easily separated. Mixing, especially evenly spreading, of solute and solvent aids and speeds the process of dissolution. Mixing can be achieved by any method, including spraying, stirring, shaking or otherwise agitating. The term "improved mixing" refers to a situation wherein the components of a mixture are more evenly distributed and not clumped together. Improved mixing may be achieved, for example, by speeding the mixing up. In the context of a solution, improved mixing results in a solution of the correct concentration because all or most of the solutes dissolve, rather than remaining as clumps or aggregated within the solvent.

The term "nozzle" as used herein refers to a fitting, usually at the end of a tube or pipe, which controls the flow of a fluid (liquid or gas) through said fitting. It may control (in speed, direction or otherwise) the entry of the fluid into a container, especially a bag. It may accelerate or decelerate the fluid. It may control the direction of the fluid. The nozzle may control the density of spray of the fluid and direction of spray of fluid from the nozzle.

The term "outlet" refers to a portion of a component through which fluid or gas can be pulled out of said component and into another component, conduit, or fluid passageway of any type. The term "pass through" refers to an action of fluid or gas passing a component positioned in a flow path. The fluid or gas can enter an inlet of the component and exit via an outlet of the component to continue the flow path.

The term "pressure" refers to a force exerted by a gas or liquid on the walls of a component, container, or conduit.

"Purified water" can be defined as water produced by distillation, deionization, reverse osmosis, or other suitable processes that remove impurities in water.

The term "reconstituting" or "re-constituting as used herein refers to restore to an original state. In the context of the solutions of the invention, this term is used to mean returning a solution to its original form, before liquids were removed, or in creating a solution from concentrated liquid and/or dried components.

The term "reconstituted" or "re-constituted" refers to an item, product or solution produced after reconstituting as defined above.

The term "solute" as used herein refers to a substance dissolved in a solution. The solute may comprise one or more chemicals or compounds. Solutes as used herein, before being dissolved in a solvent, may exist as entirely or partially dried, gels, concentrated liquids, pellets or powders, or any combination of these forms.

The term "solution" as used herein refers to a liquid mixture in which a solute is uniformly distributed within a solvent. A "solution for dialysis" may refer to any solution which can be used in any form of dialysis. It is envisaged that the solutions described herein contain specific concentrations of solutes.

The term "solvent" as used herein refers to a substance which dissolves a solute. Preferably the solvent is a liquid, and most preferably it is water.

As used herein, the "Venturi effect" reduction in fluid pressure, and increase in velocity, caused by a fluid flowing through a constricted section of a tube or pipe. Preferably the constriction is caused by a reduction in diameter of the tube or pipe.

The term "water purification unit" refers to any single component or system which can produce purified water (as defined above) from tap water or other water containing any type of impurity.

The term "container identification indicia" refers to any information relating to the container itself or the content of the container, including but not limited to, the size and weight of the container and/or its contents, the expiry date of its contents, the constituents of its contents, and the concentration of the contents.

### System

The present invention provides a dialysis machine or system with reading technology to collect data relating to various supply constituents that are inserted into the machine. This is achieved by the provision of readable tags on the constituent containers, such as the dialysate bags, which feeds information to the machine relating to their content. For example, the information stored on a tag associated with a constituent container may include at least one of the following information: content; expiry date; weight and concentration. The dialysis machine is provided with appropriate reading technology integrated into the machine to enable automatic reading of the information stored on the tag and optionally, uses this feedback to adjust an operating mode of the machine.

The present invention is particularly applicable to an improved automated peritoneal dialysis machine that includes a preparator and cycler for *in situ* preparation of peritoneal dialysis fluid (PDF) for delivery and drainage from a patient. The tagging of the powder constituent containers (generally in the form of flexible bags) assists in the creation and correct proportioning of the constituents in the PDF.

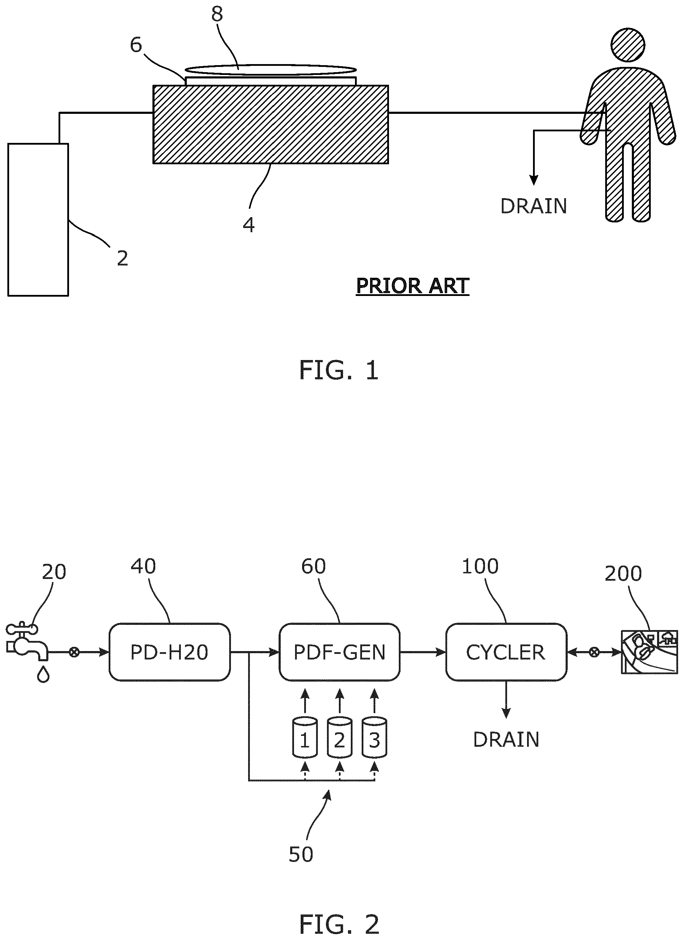

The basic concept of an automated peritoneal dialysis machine according to the prior art is shown in FIG. 1 of the accompanying drawings. The machine is fluidly connected to a patient via a catheter and transfer set and the machine has a container or bag 2 with a fresh solution supply of peritoneal dialysis fluid (PDF) which is delivered to a heating bag 8 placed on a heat source, such as a heating plate 6 to warm the PDF prior to delivery to the patient. The fluid remains in the patient for a predetermined dwell time and then removed from the patient, normally being collected in a drainage bag. The parts are fluidly connectable to provide fluid paths through the system creating a fluid system which is controlled by a controller 4. The machine includes a pump for transport of the fluid to and away from the patient, valves to control the fluid flows, a heat source to warm the PDF prior to its introduction into the patient and sensors connected to a controller to monitor and control the process.

The coupling of the fluid paths to the machine may be conveniently achieved by the provision of a disposable cassette which includes connections to each fluid path. The cassette provides sterile, disposable fluid pathways and generally includes a pump region, valve region, a sensor region and optionally a heating region. The cassette has connections for coupling with the dialysate container, the catheter unit and the drainage outflow.

The machine controls and monitors the introduction of fresh PDF into the abdominal cavity via the inlet and transfer set and the elimination of waste fluid via the transfer set and outlet. Sequential treatment cycles are carried out on a patient, normally overnight. However, the production of sufficient PDF for these cycles, particularly in a home-based setting, can be problematic. The incorporation of identification tags on the PDF bags according to the present invention, together with appropriate reading technology integrated into the dialysis machine enables data to be collected about fluid that is being delivered to the patient and provide appropriate feedback. This ensures the correct fluid content and concentration is delivered to the patient and that the fluid is not being administered beyond its expiry date.

The Applicant has devised an improved peritoneal dialysis machine that includes a preparator as well as a cycler to generate and supply PDF to the cycler, the PDF being prepared *in situ* from dialysate constituents and drinking water that is purified by the machine prior to mixing with the constituents. The integration of tag reading technology within the machine together with associated tags on the various containers is particularly beneficial for this type of machine. Figure 2 of the accompanying drawings provides a high level block diagram of the preparator and cycler. The system is able to generate sterile and apyrogenic PDF inline and on demand, using powder and/or liquid dialysate chemical ingredients and water purified by the system. The cycler then warms the PDF and delivers it to the patient.

The system uses water, such as drinking water 40 from a tap 20, that is subject to a purification step for reconstituting different powders contained in containers or constituent bags 50 and then correctly proportions the concentrates to generate the peritoneal dialysis fluid 60. Each of the containers 50 is provided with an identification tag 1, 2, 3, such as a barcode or other type of readable tag, providing details relating to the content of that specific container, such as the chemical constituents within the container, their expiry date, weight and concentration. Optionally, the containers may be provided on scales to weigh their content. Drinking water is supplied to a purification system and this purified water is then moved through the powdered chemical constituents to create dissolution of the chemicals and these are then proportioned appropriately to obtain the PDF solution for passing to the cycler 100.

In an embodiment, the powder concentrates may be provided in compartmentalized constituents bag with each compartment having an inlet and outlet with a two-way valve whereby purified water can enter each compartment and is then delivered to a mixing bag containing the generated PDF prior to delivery to the cycler. In this arrangement, each compartment should include its own unique data tag, such as a barcode or other type of readable tag. The cycler then delivers fresh PDF to the patient 200 and removes waste fluid via the drain outlet. This solves major problems on automated peritoneal diffusion, creating PDF starting from drinking water and containers with powders/concentrates, providing key benefits such as a reduction of the weight of the bags/containers that the patient has to handle; significant reduction of number, volume and weight of supplies; easier operation for patient; reduced infection risk; therapy customization and GDP reduction.

The system and method of the present invention eliminates PD proportioning & Reconstitution errors due to variations in Powder filling. The readable tag including container identification indicia is applied by a manufacturer on each constituent bag. For example, during the concentrate filling process, manufacturing equipment is configured to take the weight of an empty bag and the bag once filled after dispensing of the concentrate into the bag. A data writer records the difference in weight onto the data tag of the respective bag or compartment which corresponds to the weight of the concentrate in the bag. The manufacturing equipment may also write other information such as date of manufacture, expiry date, serial no, LOT number, contents of the bag etc into the tag fixed to the bag. Once the bag is fitted within the dialysis system, the actual weight of the bag provided for dialysis is taken, for example using scales or a load cell. The difference between the data provided for a manufactured concentrate bag and the actual weight of the bag may be utilised to improve the accuracy of the PD fluid generated by the automated peritoneal dialysis machine. In this respect, the system uses the concentrate weight data to calculate an accurate amount of water to be mixed with the concentrate to get the required dilution with minimal or no errors. Thus, the present invention provides an overall reduction in error of ions, dextrose and sodium content in the final PD fluid and ensures that the parameters are kept within the pharmacopeia limits.

The combined peritoneal dialysis machine having a preparator as well as a cycler must be able to reconstitute powders (dissolve them in a liquid) and proportion them to create a pharma fluid suitable for delivery to a patient. Ideally, bags are provided that are equipped with nozzles to generate a turbulent flow, particularly using the Venturi effect, necessary to dissolve the powder and a hydraulic machine generates the flow to move different amounts of fluid. The system has a reconstitution part wherein the powders are dissolved and a proportioning part to proportion the right amount of concentrate formed from the dissolved powders.

The reconstitution part of the system includes the bags filled with the powders required to form the correct concentrate, such as dextrose, magnesium and calcium and lactate bicarbonate and sodium chloride. Electro valves are provided on entry and exit to the bags to deviate the flow path. A high pressure flow is required to ensure that the powders are completely dissolved, and a heater may be provided to aid dissolution and to raise the temperature of the resultant PDF. The proportioning part of the system requires a more accurate flow rate adjuster, such as a peristaltic pump, to move precise amounts of liquid in order to proportion the correct amount of concentrate, together with a scale system to measure the mass of the three solutions in order to proportion the right amount of concentrate.

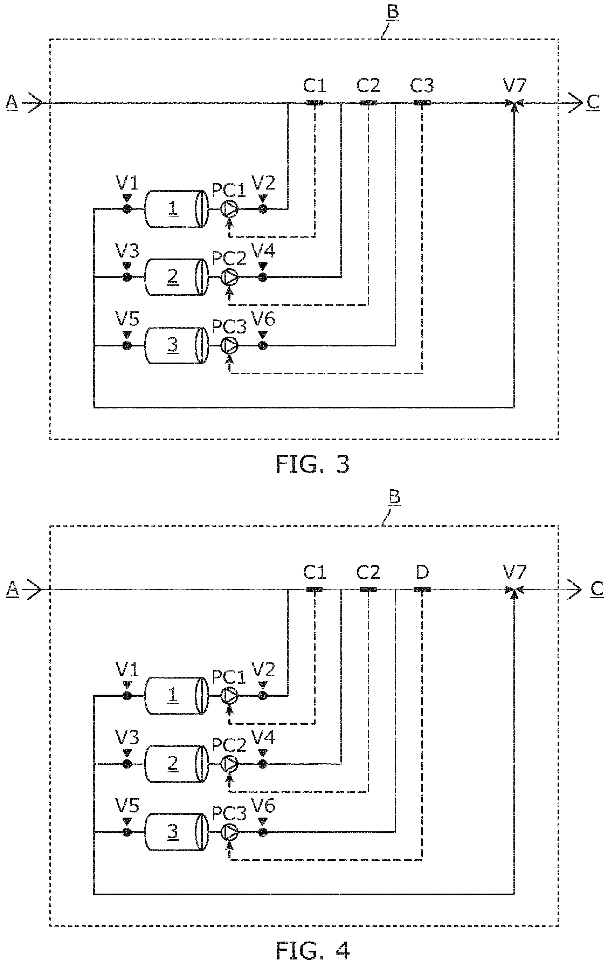

Figures 3 to 5 of the accompanying drawings provide three embodiments of fluid flow diagrams for the PD fluid generator part B of the dialysis system, each system incorporating unique identification tags 1, 2, 3, on the constituent bags. Figure 3 illustrates purified water A entering each of the constituent bags to produce concentrate that is then correctly proportioned to provide the PDF for passing through the cycler C. This embodiment implements the concentrate proportioning with conductive cells C1, C2 and C3 in a closed loop on volumetric pumps PC1, PC2, PC3. Each volumetric pump pushes its related concentrate into the main fluid flow. The concentrate's conductivity has been measured by its conductive cell which provides feedback to control the flowrate of the pump to correctly proportion the concentrate in the main flow. The fluid flow system is provided with appropriate two-way valves V1 to V6 and a three-way valve V7 to allow the required flow of fluids through the system. A similar arrangement is provided with the fluid flow diagram of Figure 4 except constituent bag with identification tag 3 contains dextrose and the conductivity cell associated with this stream is replaced with a dextrose sensor D.

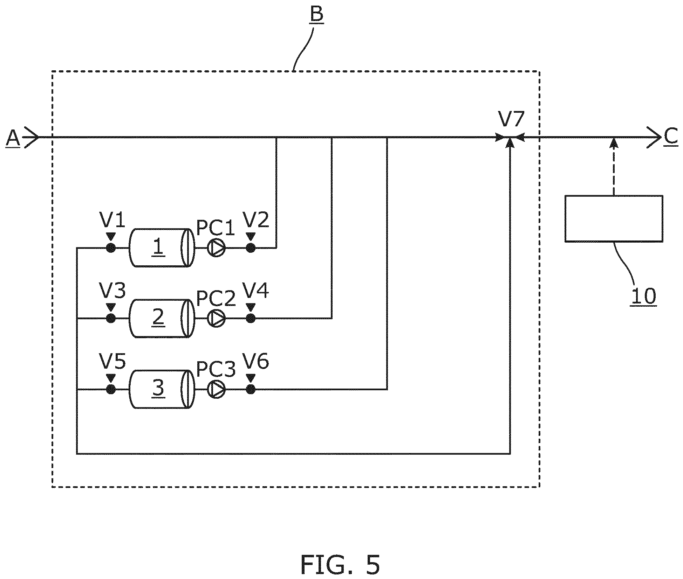

Figure 5 of the accompanying drawings illustrates yet another fluid flow diagram for the PD fluid generator B. In this embodiment, the conductive cells are not required and instead the concentrates are apportioned in the main flow for the PDF generation by highly accurate volumetric pumps, using a defined, accurate and fixed flow rate. The generated PDF is monitored using one or more of a flow meter, load cell or conductive sensor 10. In this embodiment no mixing bag is required to mix the concentrate into the required solution due to highly optimized and accurate control and mixing of the powder with the purified water. Thus, this system may be regulated by a controller to provide online in demand proportioning of the constituents making up the PDF.

One of the challenges with this system is to ensure that the correct proportion of water and concentrates are mixed to obtain the desired PDF, ideally being provided in a completely automated way. This is achieved by a volumetric approach that controls the hydraulic flow paths that introduce purified water to the powder concentrates, provide mixing of the concentrate to form the PDF and delivery of the freshly made PDF to the patient. In particular, different configurations of hydraulic flow/pressure generators are provided in the fluid paths to provide optimization of the flow of water through the fluid system to create the correct powder concentrates and subsequent peritoneal dialysis fluid for cycling. In preferred embodiments of the invention, some or all of the particular configurations are provided within one or more disposable cassette(s) having the appropriate inlet and outlet connections, a flow generator and a plurality of valves provided in the various fluid paths. For example, a cassette for the preparator may include an inlet connection, an outlet connection, at least one connector to the pure water source and a connector to each of the concentrate containers.

The control of fluid flow through this type of system requires appropriate data collection and processing to ensure correct proportioning of the constituents. It is also important to ensure that the correct constituents and viable constituents are used for the generation of the PDF. The incorporation of readable tags to identify the content of each specific container/bag, together with appropriate reading technology integrated into the dialysis machine enables data to be collected about fluid that is being delivered to the patient and provide appropriate feedback. This enables automatic collection of data about the dialysate constituents and their containers, avoiding the manual entering of information on the device by users. Furthermore, this ability to automatically read information about each constituent enables the system to adapt its volumetric pumps to ensure the containers are provided with the correct volume of purified water to obtain homogenous dialysis concentrates with the appropriate chemical concentration to create the correct PD fluid.

The readable identification tags and reading technology within the dialysis machine may be integrated with further technology to provide feedback throughout the entire system to enable automatic adjustment of the various fluid flows to provide a desired PDF. For example, through the application of tanks and load cells, the tag's reading for a container provides the typology, weight and concentration of a specific dialysate constituent 1, 2, 3. In this manner, the system can calibrate correctly the load cells that measures the water tank weight and adapt the volumetric pumps to fill both the water tank and the dialysate constituent containers with the proper volume of produced water in order to create the correct concentrates to provide the correct PD fluid.

The present invention enables customization of dialysis therapy by linking input commands relating to the required PDF for a particular patient with the identification tags of the constituents. It is possible to automate the collection of data about the dialysate constituents which minimizes the probability of user input error and device misuse. Furthermore, the incorporation of the readable tags and reading technology into a combined peritoneal preparator and cycler machine greatly improves the accuracy of the creation of the PD fluid by correcting or avoiding any errors during dialysate constituent weight measurement, set-up and container manufacture. It also enables treatment modularity by the use of different dialysate constituent concentrations thereby personalizing dialysis therapy for each patient without changes in the device settings. The operating modes of the machine may be adapted to feedback received from the various readings provided throughout the system, such as concentration, temperature, flow rate, pressure, and treatment time.

This present invention provides an overall volume reduction in relation to the dialysis system due to the presence of reduced disposable component numbers. Furthermore, the system is more compact, providing cost savings with logistics and packaging.

It is to be appreciated that all parts of the system should be leak-proof so that fluids cannot escape. The container or bags of the system described herein may be made of any material, preferably a polymer, most preferably a plastic. The bag may be rigid or flexible. Preferably the bag is flexible, resistant and stretchable, most preferably in any direction. The bag may be made of a multi-layered material, or a single layered material.

The system preferably uses a bag containing a v-shaped, funnel shaped or cone shaped section, with the opening for the nozzle at the point of the cone. Such a shape maximises the mixing of the fluid spray leaving the nozzle with the solutes in the bag to aid dissolution. However, the bag can be replaced by a solid container, preferably shaped with a cone-shaped part in which to fit a nozzle. Said container may be a box or carton, and is preferably made of a plastic.

Examples of solutes may for example comprise the following or any combination thereof:- all electrolytes, powdered acids and bases, citric acid, sodium chloride, hydrogen carbonate, calcium and magnesium ions and salts, sodium, potassium, citric acid, sodium bicarbonate and all bicarbonate salt forms, sodium lactate, and osmotic agents such as glucose, dextrose, polydextrose, polydextrine and xylitol.

The dialysis system including optionally a cassette may include other conventional components of a dialysis machine, such as heating elements, controllers, sensors and scales. The system is particularly suitable for use in dialysis carried out at home, or another environment away from a hospital or medical practice. They may also be used in a hospital or any kind of medical practice.

One skilled in the art will understand that various combinations and/or modifications and variations can be made in the described apparatus, methods and uses depending upon the specific needs for operation. Moreover, features illustrated or described as being part of an aspect of the invention may be used in the aspect of the invention, either alone or in combination.

## Claims

1. A dialysis system comprising a fluid pumping system for forming fluid paths between a container of dialysis fluid, a dialyser and to drain wherein the system is provided with at least one flow generator, at least one readable data tag including container identification indicia associated with the container of dialysis fluid, and a reader to read data from the tag.

2. The dialysis system as claimed in claim 1 wherein the readable data tag includes data relating to the content of the container selected from at least one of the following: content data; expiry date data; weight data and concentration data.

3. The dialysis system as claimed in claim 1 or claim 2 wherein the reader is configured to intermittently or continuously read data from the at least one data tag and optionally store the data locally or remotely.

4. The dialysis system as claimed in claim 1, 2 or 3 wherein the data tag is selected from at least one of: a one or two-dimensional barcode, a passive integrated transponder tag or a miniature radiofrequency tag.

5. The dialysis system as claimed in any one of claims 1 to 4 further comprising a microprocessor configurable via the dialysis system or an external host to selectively provide reading and/or writing of the data tag.

6. The dialysis system as claimed in claim 5 wherein the microprocessor is configured to compare the data retrieved against other data stored in a database and issue commands to adjust operation of the dialysis system dependent on the data received.

7. The dialysis system as claimed in claim 6 wherein the microprocessor compares a manufacturer recorded weight of the container provided on the data tag with an actual weight of the container.

8. The dialysis system as claimed in any one of the preceding claims wherein a patient's abdomen, the peritoneum, forms the dialyser membrane and the dialysis fluid is peritoneal dialysis fluid (PDF) for introducing into a peritoneal cavity of a patient, said at least one data tag including container identification indicia being associated with the container of PDF.

9. The dialysis system as claimed in claim 8 wherein the peritoneal dialysis system comprises a peritoneal dialysis preparator and cycler, the preparator comprising a purified water source fluidly connectable to a plurality of containers each containing one or more dialysate constituents for dissolution for proportioning to form the peritoneal dialysis fluid for delivery to the cycler for subsequent delivery to and drainage from a patient, each dialysate constituent container having at least one readable data tag including container identification indicia associated with it for providing information relating to the content of that container.

10. The dialysis system as claimed in claim 9 wherein each container is fluidly connectable to a chamber for receiving a solution of the constituents for preparation of the dialysis fluid, said chamber being fluidly connectable to the cycler.

11. The dialysis system as claimed in claim 9 or 10 wherein an arrangement of hydraulic flow or pressure generators are provided throughout the fluid paths to provide optimized dissolution of the constituents, optimized mixing of the solution and/or cycling of the peritoneal dialysis fluid thus formed, the data retrieved from the data tags relating to the content of the constituent containers automatically adjusting the flow generators to provide a desired PDF concentration and flow as required for a particular dialysis patient.

12. The dialysis system as claimed in claim 9, claim 10 or claim 11 wherein three constituent containers are provided that are each fluidly connectable to the purified water source, with each fluid path having a hydraulic flow generator, each constituent container having at least one data tag for transmitting container identification indicia to a data collection system and the system including a microprocessor configurable via the dialysis system or an external host to selectively provide reading and/or writing of the data tags.

13. The dialysis system as claimed in claim 12 wherein a microprocessor stores the data retrieved and compares the data against other data stored a database, the microprocessor being configured to adjust operation of the system dependent on the data received.

14. The dialysis system as claimed in claim 13 wherein the microprocessor reads a manufacturer recorded weight of each container containing one or more dialysate constituents provided on their data tag and compares this with an actual weight of each container, whereby the microprocessor applies the actual concentrate weight data to calculate and adjust the volume of purified water to be supplied to the concentrate for correct dissolution for proportioning to form the peritoneal dialysis fluid.

15. A method of monitoring a dialysis system having fluid paths for fluid flow between a container of dialysis fluid, a dialyser and a drain, the method comprising the steps of: attaching at least one readable data tag including container identification indicia to the container and reading the data from the tag.

16. The method according to claim 15 further comprising storing the data retrieved from the tag and optionally comparing the data with data in a database.

17. The method according to claim 16 further comprising adjusting the fluid flow through the fluid paths on the basis of the retrieved data.

18. The method according to claim 15 further comprising retrieving data from multiple readable data tags provided on multiple containers having different constituents that are reconstituted and proportioned to form the dialysis fluid wherein the method includes adjusting fluid flow through the fluid paths to and from these containers on the basis of the retrieved data.

19. The method according to claim 18 further comprising retrieving data relating to a manufacturer recorded weight of the container provided on the readable data tags and comparing this data with an actual weight of each container and adjusting the fluid flow to and from these containers based on this comparison for correct proportioning to form the peritoneal dialysis fluid.
